Europäisches Patentamt

European Patent Office

Office européen des brevets

(19)

(11) **EP 1 221 612 A1**

(12) **DEMANDE DE BREVET EUROPEEN**

(43) Date de publication:
**10.07.2002 Bulletin 2002/28**

(51) Int Cl.7: **G01N 33/00**, H01H 33/56

(21) Numéro de dépôt: **02290013.8**

(22) Date de dépôt: **04.01.2002**

(84) Etats contractants désignés:
**AT BE CH CY DE DK ES FI FR GB GR IE IT LI LU
MC NL PT SE TR**
Etats d'extension désignés:
**AL LT LV MK RO SI**

(30) Priorité: **08.01.2001 FR 0100161**

(71) Demandeur: **Alstom
75116 Paris (FR)**

(72) Inventeurs:
• **Chetay, Olfa
69200 Venissieux (FR)**

• **Dupraz, Jean-Pierre
69003 Lyon (FR)**
• **Lucot, Lionel
69210 Fleurieux (FR)**
• **Taponat, Didier
69100 Villeurbanne (FR)**

(74) Mandataire: **Gosse, Michel et al
ALSTOM
Intellectual Property Department
25,avenue Kléber
75116 Paris (FR)**

(54) **Procédé pour contrôler de façon non intrusive un taux de mélange d'un mélange gazeux à au moins deux composants.**

(57)   Le procédé pour contrôler de façon non intrusive la proportion d'un composant dans un mélange de gaz à au moins deux composants contenu dans une enceinte d'un appareillage électrique, consiste à mesurer la pression (P), la température (T) et la masse volumique (Rho) du mélange gazeux à l'aide de capteurs (2,3) montés sur ladite enceinte (1) et à déterminer ladite proportion par traitement desdites valeurs de mesure dans une unité de traitement de données (4).

**EP 1 221 612 A1**

**Description**

L'invention porte sur un procédé pour contrôler de façon non intrusive un taux de mélange d'un mélange gazeux à au moins deux composants qui est sous une pression de quelques bars à l'intérieur d'une enceinte.

L'invention s'applique plus particulièrement à la surveillance du gaz d'isolation dans les appareillages électriques haute tension à isolation au gaz.

Le gaz d'isolation typiquement utilisé dans ces appareillages est de l'hexafluorure de soufre ($SF_6$). Pour lutter contre le réchauffement de la planète du à l'émission de gaz à effet de serre, la tendance actuelle est de mélanger le $SF_6$ à un autre gaz comme l'azote ($N_2$) ou le fluorure de carbone ($CF_4$). Ce type de mélange à deux composants améliore en outre les performances des appareillages électriques de coupure aux très basses températures (-50°C). Il n'est pas excluque des mélanges à plus de deux composants soient utilisés à l'évenir.

Le taux du mélange $SF_6/N_2$ ou $SF_6/CF_4$ est de l'ordre de 50/50 à 80/20 environ. Pour maintenir un pouvoir de coupure satisfaisant dans un appareillage électrique à isolation avec un mélange de gaz du type $N_2$/-$SF_6$ ou $CF_4/SF_6$, il est essentiel que la proportion de $N_2$ ou de $CF_4$ dans le mélange de gaz reste constante même en cas de fuite. En effet des pertes différentielles entre les deux composants du mélange peuvent entraîner des pertes de performances en pouvoir de coupure.

Il y a également un besoin pour les fabriquants d'appareillages électriques à isolation au gaz de spécifier avec précision le taux du mélange après le remplissage, notamment pour répondre aux exigences des conditions de qualification des appareillages.

Pour déterminer le taux de mélange ou encore la proportion d'un composant dans un mélange de gaz à deux composants, on sait utiliser la chromatographie ou des techniques acoustiques. Ces méthodes restent toutefois cantonnées à une utilisation en laboratoire et ne sont pas applicables à la surveillance sur site du gaz d'isolation dans un appareillage électrique. De plus, ces méthodes sont dites intrusives car elles nécessitent un prélèvement du mélange de gaz, ce qui n'est pas compatible avec les conditions d'exploitation des appareillages électriques à isolation au gaz.

Le but de l'invention est donc d'apporter une solution simple, peu coûteuse et non intrusive, pour contrôler avec précision la proportion d'un composant par rapport à l'autre dans un mélange de gaz à au moins deux composants.

Plus particulièrement, le but de l'invention est de pouvoir contrôler avec précision la proportion de $N_2$ ou de $CF_4$ dans un mélange de gaz $N_2/SF_6$ ou $CF_4/SF_6$ servant de gaz d'isolation pour un appareillage électrique haute tension.

Le procédé de contrôle selon l'invention utilise notamment les équations de la thermodynamique afin de déterminer la proportion d'un composant dans le mélange. En effet, il est connu que dans un mélange à au moins deux composants qui ont des masses moléculaires suffisamment différentes est entièrement déterminé par quatre grandeurs : température, pression, masse volumique et taux de mélange. Des essais ont montré qu'en utilisant des capteurs industriels pour la mesure de la température, la pression et la masse volumique, capteurs qui sont disponibles dans le commerce, il est possible d'en déduire par calcul ou par extraction dans une table le taux du mélange de façon relativement précise.

L'invention a donc pour objet un procédé pour contrôler la proportion d'un composant dans mélange gazeux à au moins deux composants contenu dans une enceinte d'un appareillage électrique, caractérisé en ce qu'il consiste à mesurer la pression, la température et la masse volumique du mélange gazeux à l'aide de capteurs montés sur ladite enceinte et à déterminer ladite proportion par traitement desdites valeurs de mesure dans une unité de traitement de données, pour permettre un contrôle non intrusif du mélange.

La mesure d'une grandeur représentative de la masse volumique du mélange de gaz peut être effectuée à l'aide d'un capteur à lames vibrantes. Ce type de capteur comporte deux lames vibrantes, l'une dans le vide, l'autre dans le mélange de gaz et restitue la différence de fréquence de battement entre les deux lames, cette différence de fréquence étant représentative de la masse volumique du mélange de gaz. La masse volumique du mélange de gaz peut encore être dérivée d'une mesure de la permittivité du mélange de gaz à l'aide d'un condensateur ou analogue bien connu de l'homme de l'art. La masse volumique du mélange de gaz peut encore être dérivée d'une mesure de l'indice de réfraction du mélange de gaz à l'aide d'un interféromètre ou analogue.

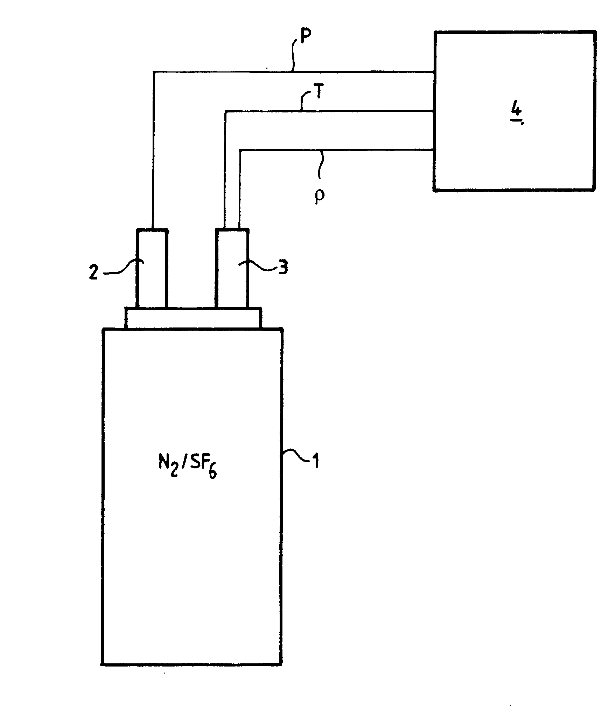

Le procédé selon l'invention est décrit ci-après en détail et illustré par la figure unique.

La figure montre de façon très schématique un système pour surveiller un mélange de gaz d'isolation à deux composants, par exemple $N_2/SF_6$, dans un disjoncteur haute tension à isolation au gaz, et contrôler en continu la proportion de $N_2$ dans le mélange de gaz.

Sur la figure, l'enceinte 1 hermétiquement close qui représente l'enceinte métallique du disjoncteur haute tension, est remplie du mélange $N_2/SF_6$ sous une pression de quelques bars, typiquement entre 4 et 8 bars. Un capteur de pression 2 et un capteur de densité 3 sont montés sur la paroi extérieure de l'enceinte 1.

Le capteur de pression 2 fournit en continu un signal P représentatif de la pression absolue du mélange de gaz dans l'enceinte. Le capteur de densité 3 fournit en continu un signal Rho représentatif de la densité du mélange de gaz et également un signal T représentatif de la température du mélange de gaz.

Ces trois signaux sont envoyés dans une unité de traitement 4 qui fournit en sortie la proportion de $N_2$ dans le mélange ou de façon analogue le taux du mélange.

Le taux de mélange, c'est à dire le rapport des pression partielles de $N_2$ et de $SF_6$ dans le mélange peut d'abord être déterminé par résolution des équations d'état thermodynamique des deux composants du mélange (équations de Beattie-Bridgeman).

Si P,T et Rho sont les variables mesurées par les capteurs et X le taux de mélange à déterminer, les équations de Beattie-Bridgeman pour un mélange à deux composants donnent les relations suivantes :

$$P \; SF_6 = A1.\rho \; SF_6 + A2.( \rho \; SF_6)^2 + A3.( \rho \; SF_6)^3$$

$$P \; N_2 = A4.\rho \; N_2$$

$$P = X. \; P \; N_2 + (1-X). \; P \; SF_6$$

$$\rho = \rho \; N_2 + \rho \; SF_6$$

où

A1,A2,A3,A4 sont des fonctions bien connues de T

$P \; SF_6$ et $P \; N_2$ sont les pressions partielles de $N_2$ et de $SF_6$

$\rho \; N_2$ et $\rho \; SF_6$ sont les masses volumiques de $N_2$ et de $SF_6$

Sur la base de ces équations, l'unité de traitement de données 4 fournit en sortie et en continu le taux de mélange X.

En variante, la proportion de $N_2$ par rapport au $SF_6$ dans le mélange de gaz peut être obtenue à partir d'une table de données préalablement constituée au cours d'une campagne d'essais. Plus particulièrement, on remplit un volume d'essai avec un mélange de gaz dont le taux de mélange est connu. On fait varier la température du volume d'essai par palier par exemple entre -40 et +60°C. On mesure pour chaque palier, la température, la pression et la masse volumique du mélange et on enregistre ces trois valeurs en correspondance avec le taux de mélange dans la table. On répète ces opérations pour différents taux de mélange. La table de données ainsi constituée est ensuite chargée et maintenue en mémoire dans l'unité 4 pour déterminer un taux de mélange en fonction des trois grandeurs mesurées que sont la température, la pression et la masse volumique.

Comme indiqué plus haut , il est possible d'utiliser un capteur à lames vibrantes qui mesure une grandeur physique représentative de la masse volumique du mélange de gaz. Dans ce cas, la température peut être dérivée du capteur de pression car les capteurs industriels modernes pour la mesure de pression délivrent à la fois la température et également la pression du gaz contrôlé.

Les mesures peuvent être avantageusement exploitées au sein d'un système de traitement de données 4 sous la forme d'un micro-ordinateur portatif ou non, installé à demeure ou de façon mobile sur l'enceinte de l'appareillage électrique. L'unité 4 peut également être un circuit électronique à microprocesseur ou à mmicrocontrôleur intégré dans un appareillage regroupant un ou plusieurs capteurs. Les capteurs peuvent être physiquement séparés, ou alors intégrés dans un même appareil de mesure multifonctionnel. Sur la figure, le capteur 3 regroupe les fonctions pression et masse volumique.

Dans le cas où l'unité de traitement de données 4 serait un micro-ordinateur mobile, il peut être avantageux que cette unité maintienne en mémoire plusieurs tables de données du type indiqué plus haut, chacune spécifique d'un mélange de gaz. En plus, l'unité 4 peut avantageusement être programmée pour dérouler des algorithmes de correction des erreurs et dérives spécifiques aux capteurs.

Le procédé selon l'invention peut donc être mis en oeuvre avec des capteurs industriels disponibles dans le commerce. Ces capteurs ont généralement une très bonne précision de mesure, typiquement meilleure que 1% ce qui implique que l'erreur relative sur le taux de mélange peut être inférieure à 1%. Avec le procédé selon l'invention, les mesures se font de façon non intrusive sans prélèvement de gaz. Ce procédé peut s'appliquer parfaitement pour une gamme de température étendue, typiquement entre -50°C et +90°C, ce qui correspond aux conditions extrêmes d'exploitation de certains appareillages électriques haute tension à isolation au gaz.

Bien entendu le procédé selon l'invention peut parfaitement s'appliquer à des mélanges de gaz à deux composants différents de $N_2/SF_6$ ou $CF_4/SF_6$ du moment que la précision des capteurs soit convenablement choisie en fonction des différences des masses moléculaires des deux composants. L'invention peut également s'appliquer à un mélange de gaz à plus de deux composants différents.

**Revendications**

1. Un procédé pour contrôler la proportion d'un composant dans un mélange gazeux à au moins deux composants contenu dans une enceinte d'un appareillage électrique, **caractérisé en ce qu'**il consiste à mesurer la pression (P), la température (T) et la masse volumique (Rho) du mélange gazeux à l'aide de capteurs (2,3) montés sur ladite enceinte (1) et à déterminer ladite proportion par traitement desdites valeurs de mesure dans une unité de traitement de données (4), pour permettre un contrôle non intrusif du mélange.

2. Le procédé selon la revendication 1, dans lequel ladite proportion d'un composant dans le mélange est calculée par l'unité de traitement de données (4) qui est programmée pour résoudre les équations d'état thermodynamique desdits composants.

3. Le procédé selon la revendication 1, dans lequel ladite proportion d'un composant dans le mélange est déterminée par l'unité de traitement de données (4) qui maintient en mémoire une table de données, cette table de données contenant plusieurs données représentatives de différentes proportions dudit composant en correspondance avec des données représentatives de différentes mesures de pression, température et masse volumique du mélange de gaz contenant ledit composant.

4. Le procédé selon la revendication 1,2 ou 3, dans lequel la masse volumique est mesurée au moyen d'un capteur à lames vibrantes.

5. Le procédé selon la revendication 1,2 ou 3, dans lequel la masse volumique est mesurée au moyen d'un condensateur dont la capacité est fonction de la permittivité du mélange de gaz.

6. Le procédé selon la revendication 1,2 ou 3, dans lequel la masse volumique est mesurée au moyen d'un interféromètre.

7. Le procédé selon la revendication 2 ou 3, dans lequel l'unité de traitement de données (4) est un micro-ordinateur.

8. Le procédé selon la revendication 2 ou 3, dans lequel l'unité de traitement de données (4) est conçue au moyen de microprocesseurs ou microcontrôleurs.

9. Un appareillage électrique comprenant une enceinte (1) contenant un mélange d'au moins deux gaz diélectriques sous pression, **caractérisé en ce que** la détermination des proportions des gaz diélectriques dans le mélange est effectuée par un procédé selon l'une des revendications 1 à 8.

10. L'appareillage électrique selon la revendication 9, dans lequel le mélange de gaz est constitué de deux composants qui sont $N_2$ et $SF_6$ ou $CF_4$ et $SF_6$.

P

T

ρ

4.

2

3

N$_2$/SF$_6$

1

**Office européen des brevets**

# RAPPORT DE RECHERCHE EUROPEENNE

Numéro de la demande

EP 02 29 0013

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| Y | FR 2 734 362 A (GEC ALSTHOM T & D SA) 22 novembre 1996 (1996-11-22) * le document en entier * | 1-3,8-10 | G01N33/00 H01H33/56 |
| Y | US 4 881 412 A (NORTHEDGE RONALD) 21 novembre 1989 (1989-11-21) * colonne 6, ligne 3 - ligne 46; figures 1-3 * | 1-3,8-10 | |
| A | DE 42 06 845 A (RATIONAL GMBH) 9 septembre 1993 (1993-09-09) * colonne 4, ligne 42 - colonne 5, ligne 50; figures 1-3 * | 1-10 | |
| A | US 6 079 217 A (JUDGE JOHN F) 27 juin 2000 (2000-06-27) * le document en entier * | 1-10 | |
| A | PATENT ABSTRACTS OF JAPAN vol. 1998, no. 08, 30 juin 1998 (1998-06-30) & JP 10 068555 A (MITSUBISHI HEAVY IND LTD), 10 mars 1998 (1998-03-10) * abrégé * | 1-10 | DOMAINES TECHNIQUES RECHERCHES (Int.Cl.7) G01N H01H |
| A | GUEHRIA F M ET AL: "COMPOSITIONAL RESERVOIR SIMULATION: A NEW, EFFICIENT, FULLY INTEGRATED SOLUTION TECHNIQUE FOR THE FLOW/THERMODYNAMIC EQUILIBRIUM EQUATIONS" SPE PROCEEDINGS, XX, XX, 20 mars 1991 (1991-03-20), pages 55-68, XP002035429 * le document en entier * | 1-10 | |
| A | EP 0 044 056 A (AKAD GORNICZO HUTNICZA) 20 janvier 1982 (1982-01-20) * le document en entier * | 1-10 | |

-/--

Le présent rapport a été établi pour toutes les revendications

| Lieu de la recherche | Date d'achèvement de la recherche | Examinateur |
|---|---|---|
| LA HAYE | 20 février 2002 | Bosma, R |

**Office européen des brevets**

## RAPPORT DE RECHERCHE EUROPEENNE

Numéro de la demande

EP 02 29 0013

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|-----------|-----------------------------------------------------|------------|------------|
| A | ZEISEL D ET AL: "A precise and robust quartz sensor based on tuning fork technology for (SF6)-gas density control" SENSORS AND ACTUATORS A, ELSEVIER SEQUOIA S.A., LAUSANNE, CH, vol. 80, no. 3, mars 2000 (2000-03), pages 233-236, XP004192111 ISSN: 0924-4247 * le document en entier * | 1-10 | |
| | | | **DOMAINES TECHNIQUES RECHERCHES (Int.Cl.7)** |

Le présent rapport a été établi pour toutes les revendications

| Lieu de la recherche | Date d'achèvement de la recherche | Examinateur |
|---------------------|-----------------------------------|-------------|
| LA HAYE | 20 février 2002 | Bosma, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**　　EP 02 29 0013

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

20-02-2002

| Document brevet cité au rapport de recherche | | Date de publication | Membre(s) de la famille de brevet(s) | | Date de publication |
|---|---|---|---|---|---|
| FR 2734362 | A | 22-11-1996 | FR | 2734362 A1 | 22-11-1996 |
| | | | AT | 186159 T | 15-11-1999 |
| | | | AU | 692652 B2 | 11-06-1998 |
| | | | AU | 4336596 A | 15-08-1996 |
| | | | BR | 9600350 A | 27-01-1998 |
| | | | CA | 2169013 A1 | 09-08-1996 |
| | | | CN | 1146012 A ,B | 26-03-1997 |
| | | | DE | 69604842 D1 | 02-12-1999 |
| | | | DE | 69604842 T2 | 04-05-2000 |
| | | | EP | 0726630 A1 | 14-08-1996 |
| | | | ES | 2138296 T3 | 01-01-2000 |
| | | | PL | 312652 A1 | 19-08-1996 |
| | | | US | 5693873 A | 02-12-1997 |
| US 4881412 | A | 21-11-1989 | GB | 2179156 A | 25-02-1987 |
| | | | AT | 58967 T | 15-12-1990 |
| | | | CA | 1285790 A1 | 09-07-1991 |
| | | | DE | 3676013 D1 | 17-01-1991 |
| | | | EP | 0213838 A1 | 11-03-1987 |
| | | | EP | 0326231 A1 | 02-08-1989 |
| | | | GB | 2213405 A ,B | 16-08-1989 |
| DE 4206845 | A | 09-09-1993 | DE | 4206845 A1 | 09-09-1993 |
| US 6079217 | A | 27-06-2000 | AU | 5250899 A | 28-02-2000 |
| | | | CN | 1274416 T | 22-11-2000 |
| | | | EP | 1019660 A1 | 19-07-2000 |
| | | | WO | 0008394 A1 | 17-02-2000 |
| JP 10068555 | A | 10-03-1998 | AUCUN | | |
| EP 0044056 | A | 20-01-1982 | PL | 225616 A1 | 18-01-1982 |
| | | | DK | 309081 A | 13-01-1982 |
| | | | EP | 0044056 A2 | 20-01-1982 |

EPO FORM P0460

Pour tout renseignement concernant cette annexe : voir Journal Officiel de l'Office européen des brevets, No.12/82